# EUROPEAN PATENT APPLICATION

(11) **EP 4 732 833 A1**
(43) Date of publication of application: **29.04.2026**
(21) Application number: 24842301.4
(22) Date of filing: 12.07.2024
(51) Int. Cl.: A61K 9/16, A61K 31/445, A61K 31/573, A61K 45/06, A61K 47/34, A61P 23/02

(54) **ROPIVACAINE MICROSPHERE AND PREPARATION METHOD THEREFOR**

(30) Priority: 19.07.2023 CN 202310893969
(71) Applicant: Bostal Drug Delivery Co., Ltd., Guangzhou, Guangdong 510320 (CN)
(72) Inventor: Tang, Chenyi, Guangdong 510320 (CN); Xie, Qian, Guangdong 510320 (CN); Li, Wei, Guangdong 510320 (CN); Wan, Xiaoli, Guangdong 510320 (CN); Liu, Rong, Guangdong 510320 (CN)
(74) Representative: Potter Clarkson
(86) International application number: PCT/CN2024/105168
(87) International publication number: WO 2025/016308

(57) **Abstract**

Disclosed in the present invention are a ropivacaine microsphere and a preparation method therefor, which belongs to the field of medicines. The ropivacaine microsphere comprises ropivacaine or a pharmaceutically acceptable salt thereof, at least one glucocorticoid or a pharmaceutically acceptable salt thereof, and at least one biocompatible and biodegradable sustained-release material at an effective amount for extending the release of a drug from a preparation, wherein the time required for the cumulative release of 80% of ropivacaine or the pharmaceutically acceptable salt thereof is more than 1.7 times the time required for the cumulative release of 80% of glucocorticoid or the pharmaceutically acceptable salt or ester thereof.

## Description

Priority claim: The present invention claims priority to Chinese patent application 202310893969.5, filed on July 19, 2023, the entire contents of which are hereby incorporated by reference.

### TECHNICAL FIELD

The present invention generally relates to the pharmaceutical field of pain management, and more specifically to implantable biodegradable microspheres for local drug delivery.

### BACKGROUND

Postoperative pain refers to acute pain that occurs immediately after surgery, typically lasting for at least 48 to 72 hours. Local administration of anesthetics is the most direct method for postoperative analgesia; however, clinically used local anesthetics have a short half-life and require frequent administration every 4 to 6 hours. Ropivacaine is a novel amide-type local anesthetic commonly used for postoperative analgesia, yet its plasma half-life is only 1.8 hours, necessitating continuous injection to achieve satisfactory analgesic effects. Currently, the marketed Ropivacaine hydrochloride injection (Naropin^{®}) provides effective analgesia for merely 6 to 8 hours, requiring frequent administration.

Clinically, there is a lack of preparations that can sustain a sufficiently prolonged anesthetic effect, thereby reducing the need for patients to undergo frequent multiple administrations.

### SUMMARY OF THE INVENTION

In one aspect, the present invention relates to a ropivacaine microsphere, which comprises ropivacaine or a pharmaceutically acceptable salt thereof, at least one glucocorticoid or a pharmaceutically acceptable salt or ester thereof, and at least one biocompatible and biodegradable sustained-release material in an effective amount for extending the release of a drug from a preparation. Wherein the time required for the cumulative release of 80% of ropivacaine or the pharmaceutically acceptable salt thereof is at least 1.7 times the time required for the cumulative release of 80% of the glucocorticoid or the pharmaceutically acceptable salt or ester thereof.

In another aspect, the present invention relates to a ropivacaine microsphere, which comprises ropivacaine or a pharmaceutically acceptable salt thereof, at least one glucocorticoid or a pharmaceutically acceptable salt or ester thereof, and at least one biocompatible and biodegradable sustained-release material in an effective amount for extending the release of a drug from a preparation. Wherein, when the cumulative release amount of ropivacaine or the pharmaceutically acceptable salt thereof reaches 10%, 20%, 30%, 40%, 50%, 60%, 70%, or 80%, the sum of the differences between the cumulative release amounts of the glucocorticoid or the pharmaceutically acceptable salt or ester thereof and ropivacaine or the pharmaceutically acceptable salt thereof is not less than 105%.

In another aspect, the present invention relates to a ropivacaine microsphere, which comprises ropivacaine or a pharmaceutically acceptable salt thereof, at least one glucocorticoid or a pharmaceutically acceptable salt or ester thereof, and at least one biocompatible and biodegradable sustained-release material in an effective amount for extending the release of a drug from a preparation. Wherein ropivacaine or the pharmaceutically acceptable salt thereof is capable of sustained release for a duration of at least 48 hours, and the glucocorticoid or the pharmaceutically acceptable salt or ester thereof is capable of sustained release for a duration of at least 24 hours.

In another aspect, the present invention relates to a ropivacaine microsphere, comprising, by mass percentage, 40-72% of ropivacaine or a pharmaceutically acceptable salt thereof, 0.1-3% of a glucocorticoid or a pharmaceutically acceptable salt or ester thereof, 20-65% of a biodegradable sustained-release material, and 0-15% of a porogen.

On the other hand, the present invention relates to a method for preparing ropivacaine microspheres, which comprises the following steps:
(1) Dissolving ropivacaine or a pharmaceutically acceptable salt thereof, at least one glucocorticoid or a pharmaceutically acceptable salt or ester thereof, at least one biocompatible and biodegradable sustained-release material in an effective amount for extending the release of a drug from a preparation, and optional polyethylene glycol in an organic solvent to obtain an oil phase;
(2) Preparing a 1% polyvinyl alcohol aqueous solution and adjusting its pH to alkaline to obtain an aqueous phase;
(3) Preparing a 0.1% polyvinyl alcohol aqueous solution and adjusting its pH to alkaline to obtain a hardening solution; and
(4) Mixing the oil phase and the aqueous phase to prepare a primary emulsion, adding the primary emulsion to the hardening solution, stirring to remove the organic solvent, then collecting, washing, and drying.

In another aspect, the present invention relates to a method for local administration to provide long-acting analgesia or nerve block, which comprises administering ropivacaine microspheres. The microspheres comprise ropivacaine or a pharmaceutically acceptable salt thereof, at least one glucocorticoid or a pharmaceutically acceptable salt or ester thereof, and at least one biocompatible and biodegradable sustained-release material in an effective amount for extending the release of a drug from a preparation. Wherein, when the cumulative release amount of ropivacaine or the pharmaceutically acceptable salt thereof reaches 10%, 20%, 30%, 40%, 50%, 60%, 70%, or 80%, the sum of the differences between the cumulative release amounts of the glucocorticoid or the pharmaceutically acceptable salt or ester thereof and ropivacaine or the pharmaceutically acceptable salt thereof is not less than 105%.

### Elaborate

In the following description, certain specific details are included to provide a comprehensive understanding of various disclosed embodiments. However, those skilled in the relevant art will recognize that embodiments can still be implemented without utilizing one or more of these specific details, and by employing other methods, components, materials, etc.

Unless otherwise required in the present invention, throughout the specification and the appended claims, the terms "including", "comprising", "containing", and "having" shall be interpreted as having an open and inclusive meaning, that is, "including but not limited to".

When used in the present invention and the appended claims, unless otherwise specified in the context, singular references without a quantity indication include plural references.

The phrases "one embodiment", "an embodiment", "in another embodiment" or "in some embodiments" mentioned throughout the specification refer to the inclusion of specific reference elements, structures, or features related to those described in at least one embodiment Therefore, the phrases "in one embodiment" or "in an embodiment" or "in another embodiment" or "in some embodiments" appearing at different locations throughout the specification do not necessarily all refer to the same embodiment. Furthermore, specific elements, structures, or features can be combined in any appropriate manner in one or multiple embodiments.

It should be understood that the use of the singular form of the article "a" (corresponding to "a", "an", and "the" in English) in the specification and appended claims of the present invention includes plural objects, unless otherwise explicitly stated in the text. Therefore, for example, the reference to a sustained-release tablet containing "a pharmaceutically acceptable excipient" includes one type of pharmaceutically acceptable excipient, or two or more types of pharmaceutically acceptable excipients.

When the term "about" refers to a quantity or numerical range, it means that the indicated quantity or numerical range is an approximate value within the variability of the experiment (or within the statistical experimental error). In the present invention, the term "about" refers to a value within 10% of the specified quantity or range, preferably within 5%.

### Definition

In the present invention, the term "ropivacaine" refers to (S)-N-(2,6-dimethylphenyl)-1-propylpiperidine-2-carboxamide.

In the present invention, the term "pharmaceutically acceptable salt" refers to those salts that maintain the biological effectiveness and properties of the free base. These salts are suitable in biological or other aspects and are formed using inorganic acids or organic acids. The inorganic acids include but are not limited to hydrochloric acid, hydrobromic acid, sulfuric acid, nitric acid, phosphoric acid, etc., the organic acids include but are not limited to acetic acid, 2,2-dichloroacetic acid, adipic acid, alginic acid, ascorbic acid, aspartic acid, benzenesulfonic acid, benzene carboxylic acid, 4-acetamidobenzoic acid, camphoric acid, camphor-10-sulfonic acid, capric acid, caproic acid, octoic acid, carbonic acid, cinnamic acid, citric acid, cyclohexylsulfamic acid, dodecylsulfuric acid, ethane-1,2-disulfonic acid, ethanesulfonic acid, 2-hydroxyethanesulfonic acid, formic acid, fumaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid, etc.

In the present invention, the term "microspheres" refers to tiny spherical entities formed by the dissolution and/or dispersion of drugs, which are encapsulated and/or adsorbed within a biocompatible and biodegradable polymer matrix. It also refers to microcapsules.

In the present invention, the term "poly(lactic-co-glycolic acid) (PLGA)" refers to a polymer obtained through polycondensation using glycolic acid and lactic acid as monomers, or a polymer obtained through ring-opening polymerization using lactide and glycolide as monomers, also known as DL-lactic and glycolic acids copolymer.

### DESCRIPTION OF THE DRAWINGS

Various embodiments of the present invention will be described below with reference to the accompanying drawings:
Figure 1 shows the cumulative release curve of ropivacaine-dexamethasone acetate microspheres in Example 1 of the present invention;
Figure 2 illustrates the thermal pain threshold curve of ropivacaine-dexamethasone acetate microspheres in rats according to Example 1 of the present invention;
Figure 3 shows the cumulative release curve of ropivacaine-dexamethasone acetate microspheres in Example 2 of the present invention;
Figure 4 illustrates the thermal pain threshold curve of ropivacaine-dexamethasone acetate microspheres in rats according to Example 2 of the present invention;
Figure 5 shows the cumulative release curve of ropivacaine-dexamethasone acetate microspheres in Example 3 of the present invention;
Figure 6 illustrates the thermal pain threshold curve of ropivacaine-dexamethasone acetate microspheres in rats according to Example 3 of the present invention;
Figure 7 shows the cumulative release curve of ropivacaine-dexamethasone acetate microspheres in Example 4 of the present invention;
Figure 8 illustrates the thermal pain threshold curve of ropivacaine-dexamethasone acetate microspheres in rats according to Example 4 of the present invention;
Figure 9 shows the cumulative release curve of ropivacaine-dexamethasone acetate microspheres in Example 5 of the present invention;
Figure 10 illustrates the thermal pain threshold curve of ropivacaine-dexamethasone acetate microspheres in rats according to Example 5 of the present invention;
Figure 11 shows the cumulative release curve of ropivacaine-dexamethasone acetate microspheres in Example 6 of the present invention;
Figure 12 illustrates the thermal pain threshold curve of ropivacaine-dexamethasone acetate microspheres in rats according to Example 6 of the present invention;
Figure 13 shows the cumulative release curve of ropivacaine-dexamethasone acetate microspheres in Example 7 of the present invention;
Figure 14 shows the paw withdrawal threshold curve of ropivacaine-dexamethasone acetate microspheres in Example 7 of the present invention;
Figure 15 shows the thermal withdrawal latency curve of ropivacaine-dexamethasone acetate microspheres in Example 7 of the present invention;
Figure 16 shows the cumulative release curve of ropivacaine-dexamethasone acetate microspheres in Example 8 of the present invention;
Figure 17 shows the cumulative release curve of ropivacaine-dexamethasone acetate microspheres in Example 9 of the present invention;
Figure 18 shows the cumulative release curve of ropivacaine-dexamethasone acetate microspheres in Example 10 of the present invention;
Figure 19 shows the cumulative release curve of ropivacaine and dexamethasone microspheres in Example 11 of the present invention;
Figure 20 shows the cumulative release curve of ropivacaine and dexamethasone microspheres in Example 12 of the present invention;
Figure 21 shows the cumulative release curve of ropivacaine-dexamethasone acetate microspheres in Comparative Example 1 of the present invention;
Figure 22 illustrates the thermal pain threshold curve of ropivacaine-dexamethasone acetate microspheres in rats according to Comparative Example 1 of the present invention;
Figure 23 shows the cumulative release curve of ropivacaine-dexamethasone acetate microspheres in Comparative Example 2 of the present invention;
Figure 24 shows the cumulative release curve of ropivacaine-dexamethasone acetate microspheres in Comparative Example 3 of the present invention;
Figure 25 shows the cumulative release curve of ropivacaine-dexamethasone acetate microspheres in Comparative Example 4 of the present invention.

### DETAILED DESCRIPTION

On the one hand, the present invention relates to a ropivacaine microsphere, which comprises ropivacaine or a pharmaceutically acceptable salt thereof, at least one glucocorticoid or a pharmaceutically acceptable salt or ester thereof, and at least one biocompatible and biodegradable sustained-release material in an effective amount for extending the release of a drug from a preparation. Wherein the time required for the cumulative release of 80% of ropivacaine or the pharmaceutically acceptable salt thereof is at least 1.7 times the time required for the cumulative release of 80% of the glucocorticoid or the pharmaceutically acceptable salt or ester thereof.

In some embodiments, the time required for the cumulative release of 80% of ropivacaine or the pharmaceutically acceptable salt thereof in the ropivacaine microspheres of the present invention is at least 1.8 times, at least 1.9 times, at least 2.0 times, at least 2.1 times, at least 2.2 times, at least 2.3 times, at least 2.4 times, at least 2.5 times, at least 2.6 times, at least 2.7 times, at least 2.8 times, at least 2.9 times, at least 3.0 times, at least 3.1 times, at least 3.2 times, at least 3.3 times, at least 3.4 times, at least 3.5 times, at least 3.6 times, at least 3.7 times, at least 3.8 times, at least 3.9 times, at least 4.0 times, at least 4.1 times, at least 4.2 times, at least 4.3 times, at least 4.4 times, at least 4.5 times, at least 4.6 times, at least 4.7 times, at least 4.8 times, at least 4.9 times, or at least 5.0 times the time required for the cumulative release of 80% of a glucocorticoid or the pharmaceutically acceptable salt or ester thereof.

In some embodiments, the cumulative release amount of ropivacaine or the pharmaceutically acceptable salt thereof from the ropivacaine microspheres of the present invention over 24 hours is not less than 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64% or 65%.

In some embodiments, ropivacaine or pharmaceutically acceptable salt thereof in the ropivacaine microspheres of the present invention is capable of sustained release for a duration of at least 48 hours, 50 hours, 52 hours, 54 hours, 56 hours, 58 hours, 60 hours, 62 hours, 64 hours, 66 hours, 68 hours, 70 hours, 72 hours, 74 hours, 76 hours, 78 hours, 80 hours, 82 hours, 84 hours, 86 hours, 88 hours, 90 hours, 92 hours, 94 hours, 96 hours, 98 hours, 100 hours, 102 hours, 104 hours, 106 hours, 108 hours, 110 hours, 112 hours, 114 hours, 116 hours, 118 hours, 120 hours, 122 hours, 124 hours, 126 hours, 128 hours, 130 hours, 132 hours, 134 hours, 136 hours, 138 hours, 140 hours, 142 hours, 144 hours, 146 hours, 148 hours, 150 hours, 152 hours, 154 hours, 156 hours, 158 hours, 160 hours, 162 hours, 164 hours, 166 hours, 168 hours, 170 hours, 172 hours, 174 hours, 176 hours, 178 hours, 180 hours, 182 hours, 184 hours, 186 hours, 188 hours, 190 hours, 195 hours, 200 hours, 205 hours, 210 hours, 215 hours, 220 hours, 225 hours, 230 hours, 235 hours, 240 hours, 245 hours, 250 hours, 255 hours, 260 hours, 265 hours, 270 hours, 275 hours, 280 hours, 285 hours, 290 hours, 295 hours, 300 hours, 305 hours, 310 hours, 315 hours, 320 hours, 325 hours, 330 hours, 335 hours, 340 hours, 345 hours, 350 hours, 355 hours, or 360 hours.

In some embodiments, the glucocorticoid or the pharmaceutically acceptable salt or ester thereof in the ropivacaine microspheres of the present invention is capable of sustained release for a duration of at least 24 hours, 25 hours, 26 hours, 27 hours, 28 hours, 29 hours, 30 hours, 31 hours, 32 hours, 33 hours, 34 hours, 35 hours, 36 hours, 37 hours, 38 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours, 61 hours, 62 hours, 63 hours, 64 hours, 65 hours, 66 hours, 67 hours, 68 hours, 69 hours, 70 hours, 71 hours, 72 hours, 73 hours, 74 hours, 75 hours, 76 hours, 77 hours, 78 hours, 79 hours, 80 hours, 81 hours, 82 hours, 83 hours, 84 hours, 85 hours, 86 hours, 87 hours, 88 hours, 89 hours, 90 hours, 91 hours, 92 hours, 93 hours, 94 hours, 95 hours, 96 hours, 97 hours, 98 hours, 99 hours or 100 hours.

In some embodiments, exemplary examples of glucocorticoids or pharmaceutically acceptable salts or esters thereof that can be used in the present invention include, but are not limited to, cortisone, hydrocortisone, prednisone, prednisolone, methylprednisolone, deflazacort, fludrocortisone, triamcinolone, betamethasone, dexamethasone, triamcinolone acetonide, methylprednisolone, paramethasone, beclomethasone, betamethasone, or flunisolide, or pharmaceutically acceptable salts or esters thereof.

In some embodiments, exemplary examples of glucocorticoids or pharmaceutically acceptable salts or esters thereof that can be used in the present invention include, but are not limited to, dexamethasone, dexamethasone acetate, dexamethasone sodium phosphate, dexamethasone palmitate, betamethasone, and betamethasone dipropionate.

In some embodiments, exemplary examples of biocompatible and biodegradable sustained-release materials that can be used to form the microspheres of the present invention include, but are not limited to, polyamide, polyamine, poly(alkylene oxalate), polyanhydride, polyamidoester, poly(ether-ester) copolymer, polycarbonate, polyorthoester, polylactide, polyglycolide, polylactic acid, poly(lactic-co-glycolic acid) copolymer, polyorthoester, polycaprolactone, polyphosphazene, polysaccharide, protein polymer, polysaccharide derivative, soluble derivative of protein polymer, polypeptide, polyester, polyorthoester, and copolymers, block copolymers, homopolymers, mixtures, and compositions thereof.

In some embodiments, exemplary examples of biocompatible and biodegradable sustained-release materials that can be used to form the microspheres of the present invention include, but are not limited to, poly(lactic-co-glycolic acid) copolymers with a molar ratio of lactic acid (lactide) to glycolic acid (glycolide) ranging from about 50:50 to about 95:5, preferably 50:50, 55:50, 60:50, 65:50, 70:50, 75:50, 80:50, 85:50, 90:50, 95:50, 50:45, 55:45, 60:45, 65:45, 70:45, 75:45, 80:45, 85:45, 90:45, 95:45, 50:40, 55:40, 60:40, 65:40, 70:40, 75:40, 80:40, 85:40, 90:40, 95:40, 50:35, 55:35, 60:35, 65:35, 70:35, 75:35, 80:35, 85:35, 90:35, 95:35, 50:30, 55:30, 60:30, 65:30, 70:30, 75:30, 80:30, 85:30, 90:30, 95:30, 50:25, 55:25, 60:25, 65:25, 70:25, 75:25, 80:25, 85:25, 90:25, 95:25, 50:20, 55:20, 60:20, 65:20, 70:20, 75:20, 80:20, 85:20, 90:20, 95:20, 50:15, 55:15, 60:15, 65:15, 70:15, 75:15, 80:15, 85:15, 90:15, 95:15, 50:10, 55:10, 60:10, 65:10, 70:10, 75:10, 80:10, 85:10, 90:10, 95:10, 50:5, 55:5, 60:5, 65:5, 70:5, 75:5, 80:5, 85:5, 90:5, 95:5.

In some embodiments, the poly(lactic-co-glycolic acid) copolymer that can be used to form the microspheres of the present invention has an intrinsic viscosity of about 0.12 dl/g to 0.21 dl/g. Preferably, the intrinsic viscosity is 0.12 dl/g, 0.13 dl/g, 0.14 dl/g, 0.15 dl/g, 0.16 dl/g, 0.17 dl/g, 0.18 dl/g, 0.19 dl/g, 0.20 dl/g, or 0.21 dl/g.

In some embodiments, the preferred grade of poly(lactic-co-glycolic acid) copolymer that can be used to form the microspheres of the present invention is Ashland/7525.

In some embodiments, the ropivacaine microspheres of the present invention further comprise a porogen.

In some embodiments, exemplary examples of porogens that can be used in the present invention include, but are not limited to, amphiphilic polymers, low molecular weight monohydric alcohols, low molecular weight polyhydric alcohols, and mixtures thereof.

In some embodiments, exemplary examples of amphiphilic polymers that can be used in the present invention include, but are not limited to, polyethylene glycol, polyvinyl ether, polyethyleneimine, polyvinyl pyrrolidone, polyacrylamide polymers, polyacrylic acid, polystyrene sulfonate salt, and mixtures thereof.

In some embodiments, the polyethylene glycol (PEG) that can be used in the present invention has a relative molecular weight of about 100 to 10,000; preferably 100, 150, 200, 250, 300, 250, 300, 350, 400, 450, 500, 550, 600, 650, 700, 750, 800, 850, 900, 950, 1,000, 1,050, 1,100, 1,150, 1,200, 1,250, 1,300, 1,350, 1,400, 1,450, 1,500, 1,550, 1,600, 1,650, 1,700, 1,750, 1,800, 1,850, 1,900, 1,950, 2,000, 2,500, 3,000, 3,500, 4,000, 4,500, 5,000, 5,500, 6,000, 6,500, 7,000, 7,500, 8,000, 8,500, 9,000, 9,500, 10,000.

In some embodiments, exemplary examples of low molecular weight monohydric alcohols that can be used in the present invention include, but are not limited to, methanol, ethanol, benzyl alcohol, and mixtures thereof.

In some embodiments, exemplary examples of low molecular weight polyhydric alcohols that can be used in the present invention include, but are not limited to, sorbitol, propylene glycol, glycerol, and mixtures thereof.

In some embodiments, the drug loading of ropivacaine or pharmaceutically acceptable salt thereof in the ropivacaine microspheres of the present invention is about 5%, 10%, 15%, 20%, 25%, 30%, 35%, 40%, 45%, 50%, 55%, 60%, 65%, or 70%.

In some embodiments, the drug loading of the glucocorticoid or pharmaceutically acceptable salt or ester thereof in the ropivacaine microspheres of the present invention is about 0.25%, 0.50%, 0.75%, 1.00%, 1.25%, 1.50%, 1.75%, 2.00%, 2.25%, 2.50%, 2.75%, 3.00%, 3.25%, 3.50%, 3.75%, 4.00%, 4.25%, 4.50%, 4.75%, 5.00%, 5.25%, 5.50%, 5.75%, 6.00%, 6.25%, 6.50%, 6.75%, 7.00%, 7.25%, 7.50%, 7.75%, 8.00%, 8.25%, 8.50%, 8.75%, 9.00%, 9.25%, 9.50%, 9.75% or 10.00%.

In some embodiments, the mass percentage content of the porogen in the ropivacaine microspheres of the present invention is about 0.20%, 0.40%, 0.60%, 0.80%, 1.00%, 1.20%, 1.40%, 1.60%, 1.80%, 2.00%, 2.20%, 2.40%, 2.60%, 2.80%, 3.00%, 3.20%, 3.40%, 3.60%, 3.80% or 4.00%.

In some embodiments, the ratio of the drug loading of ropivacaine or pharmaceutically acceptable salt thereof to the drug loading of glucocorticoid or pharmaceutically acceptable salt or ester thereof in the ropivacaine microspheres of the present invention is 30:1 to 500:1; preferably 30-32:1, 32-34:1, 34-36:1, 36-38:1, 38-40:1, 40-42:1, 42-44:1, 44-46:1, 46-48:1, 48-50:1, 50-52:1, 52-54:1, 54-56:1, 56-58:1, 58-59:1, 59-61:1, 61-63:1, 63-65:1, 65-67:1, 67-69:1, 69-71:1, 71-73:1, 73-75:1, 75-77:1, 77-79:1, 79-81:1, 81-83:1, 83-85:1, 85-87:1, 87-89:1, 89-91:1, 91-93:1, 93-95:1, 95-97:1, 97-99:1, 99-101:1, 101-103:1, 103-105:1, 105-107:1, 107-109:1, 111-113:1, 113-115:1, 115-117:1, 117-119:1, 121-123:1, 123-125:1, 125-127:1, 127-129:1, 129-131:1, 131-133:1, 133-135:1, 135-137:1, 137-139:1, 141-143:1, 143-145:1, 145-147:1, 147-149:1, 151-153:1, 153-155:1, 155-157:1, 157-159:1, 159-161:1, 161-163:1, 163-165:1, 165-167:1, 167-169:1, 169-171:1, 171-173:1, 173-175:1, 175-177:1, 177-179:1, 179-181:1, 181-183:1, 183-185:1, 185-187:1, 187-189:1, 189-191:1, 191-193:1, 193-195:1, 195-197:1, 197-199:1, 199-201:1, 201-203:1, 203-205:1, 205-207:1, 207-209:1, 211-213:1, 213-215:1, 215-217:1, 217-219:1, 219-221:1, 221-223:1, 223-225:1, 225-227:1, 227-229:1, 231-233:1, 233-235:1, 235-237:1, 237-239:1, 241-243:1, 243-245:1, 245-247:1, 247-249:1, 249-251:1, 251-253:1, 253-255:1, 255-257:1, 257-259:1, 259-261:1, 261-263:1, 263-265:1, 265-267:1, 267-269:1, 269-271:1, 271-273:1, 273-275:1, 275-277:1, 277-279:1, 279-281:1, 281-283:1, 283-285:1, 285-287:1, 287-289:1, 289-291:1, 291-293:1, 293-295:1, 295-297:1, 297-299:1, 299-301:1, 301-303:1, 303-305:1, 305-307:1, 307-309:1, 309-311:1, 311-313:1, 313-315:1, 315-317:1, 317-319:1, 319-321:1, 321-323:1, 323-325:1, 325-327:1, 327-329:1, 329-331:1, 331-333:1, 333-335:1, 335-337:1, 337-339:1, 339-341:1, 341-343:1, 343-345:1, 345-347:1, 347-349:1, 349-351:1, 351-353:1, 353-355:1, 355-357:1, 357-359:1, 359-361:1, 361-363:1, 363-365:1, 365-367:1, 367-369:1, 369-371:1, 371-373:1, 373-375:1, 375-377:1, 377-379:1, 379-381:1, 381-383:1, 383-385:1, 385-387:1, 387-389:1, 389-391:1, 391-393:1, 393-395:1, 395-397:1, 397-399:1, 399-401:1, 401-403:1, 403-405:1, 405-407:1, 407-409:1, 409-411:1, 411-413:1, 413-415:1, 415-417:1, 417-419:1, 419-321:1, 421-423:1, 423-425:1, 425-427:1, 427-429:1, 429-431:1, 431-433:1, 433-335:1, 435-437:1, 437-439:1, 439-441:1, 441-443:1, 443-445:1, 445-447:1, 447-449:1, 449-451:1, 451-453:1, 453-455:1, 455-457:1, 457-459:1, 459-461:1, 461-463:1, 463-465:1, 465-467:1, 467-469:1, 469-471:1, 471-473:1, 473-475:1, 475-477:1, 477-479:1, 479-481:1, 481-483:1, 483-485:1, 485-487:1, 487-489:1, 489-491:1, 491-493:1, 493-495:1, 495-497:1, 497-499:1, 499-500:1.

In some embodiments, the ropivacaine microspheres of the present invention have a particle size D10 of about 5µm, 6µm, 7µm, 8µm, 9µm, 10µm, 11µm, 12pm, 13µm, 14µm, 15µm, 16µm, 17µm, 18µm, 19µm, 20µm, 21µm, 22µm, 23µm, 24µm, 25µm, 26µm, 27µm, 28µm, 29µm, 30µm, 31µm, 32µm, 33µm, 34µm, 35µm, 36µm, 37µm, 38µm, 39µm, 40µm, 41µm, 42µm, 43µm, 44µm, 45µm, 46µm, 47µm, 48µm, 49µm, or 50µm.

In some embodiments, the ropivacaine microspheres of the present invention have a particle size D50 of about 10µm, 11µm, 12µm, 13µm, 14µm, 15µm, 16µm, 17µm, 18µm, 19µm, 20µm, 21µm, 22µm, 23µm, 24µm, 25µm, 26µm, 27µm, 28µm, 29µm, 30µm, 31µm, 32µm, 33µm, 34µm, 35µm, 36µm, 37µm, 38µm, 39µm, 40µm, 41µm, 42µm, 43µm, 44µm, 45µm, 46µm, 47µm, 48µm, 49µm, 50µm, 51µm, 52µm, 53µm, 54µm, 55µm, 56µm, 57µm, 58µm, 59µm, 60µm, 61µm, 62µm, 63µm, 64µm, 65µm, 66µm, 67µm, 68µm, 69µm, or 70µm.

In some embodiments, the ropivacaine microspheres of the present invention have a particle size D90 of about 20µm, 21µm, 22µm, 23µm, 24µm, 25µm, 26µm, 27µm, 28µm, 29µm, 30µm, 31µm, 32µm, 33µm, 34µm, 35µm, 36µm, 37µm, 38µm, 39µm, 40µm, 41µm, 42µm, 43µm, 44µm, 45µm, 46µm, 47µm, 48µm, 49µm, 50µm, 51µm, 52µm, 53µm, 54µm, 55µm, 56µm, 57µm, 58µm, 59µm, 60µm, 61µm, 62µm, 63µm, 64µm, 65µm, 66µm, 67µm, 68µm, 69µm, 70µm, 71µm, 72µm, 73µm, 74µm, 75µm, 76µm, 77µm, 78µm, 79µm, 80µm, 81µm, 82µm, 83µm, 84µm, 85µm, 86µm, 87µm, 88µm, 89µm, 90µm, 91µm, 92µm, 93µm, 94µm, 95µm, 96µm, 97µm, 98µm, 99µm or 100µm.

On the other hand, the present invention relates to a ropivacaine microsphere, which comprises ropivacaine or a pharmaceutically acceptable salt thereof, at least one glucocorticoid or a pharmaceutically acceptable salt or ester thereof, and at least one biocompatible and biodegradable sustained-release material in an effective amount for extending the release of a drug from a preparation. Wherein when the cumulative release amount of ropivacaine or the pharmaceutically acceptable salt thereof reaches 10%, 20%, 30%, 40%, 50%, 60%, 70%, or 80%, the sum of the differences between the cumulative release amounts of the glucocorticoid or the pharmaceutically acceptable salt or ester thereof and ropivacaine or the pharmaceutically acceptable salt thereof is not less than 105%.

In some embodiments, when the cumulative release amount of ropivacaine or pharmaceutically acceptable salt thereof from the ropivacaine microspheres of the present invention reaches 10%, 20%, 30%, 40%, 50%, 60%, 70%, or 80%, the sum of the differences between the cumulative release amounts of the glucocorticoid or the pharmaceutically acceptable salt or ester thereof and ropivacaine or the pharmaceutically acceptable salt thereof is not less than 110%, 115%, 120%, 125%, 130%, 135%, 140%, 145%, 150%, 155%, 160%, 165%, 170%, 175%, 180%, 185%, 190%, 195% or 200%.

In some embodiments, the cumulative release amount of ropivacaine or pharmaceutically acceptable salt thereof from the ropivacaine microspheres of the present invention over 24 hours is not less than 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64% or 65%.

In some embodiments, ropivacaine or pharmaceutically acceptable salt thereof in the ropivacaine microspheres of the present invention is capable of sustained release for a duration of at least 48 hours, 50 hours, 52 hours, 54 hours, 56 hours, 58 hours, 60 hours, 62 hours, 64 hours, 66 hours, 68 hours, 70 hours, 72 hours, 74 hours, 76 hours, 78 hours, 80 hours, 82 hours, 84 hours, 86 hours, 88 hours, 90 hours, 92 hours, 94 hours, 96 hours, 98 hours, 100 hours, 102 hours, 104 hours, 106 hours, 108 hours, 110 hours, 112 hours, 114 hours, 116 hours, 118 hours, 120 hours, 122 hours, 124 hours, 126 hours, 128 hours, 130 hours, 132 hours, 134 hours, 136 hours, 138 hours, 140 hours, 142 hours, 144 hours, 146 hours, 148 hours, 150 hours, 152 hours, 154 hours, 156 hours, 158 hours, 160 hours, 162 hours, 164 hours, 166 hours, 168 hours, 170 hours, 172 hours, 174 hours, 176 hours, 178 hours, 180 hours, 182 hours, 184 hours, 186 hours, 188 hours, 190 hours, 195 hours, 200 hours, 205 hours, 210 hours, 215 hours, 220 hours, 225 hours, 230 hours, 235 hours, 240 hours, 245 hours, 250 hours, 255 hours, 260 hours, 265 hours, 270 hours, 275 hours, 280 hours, 285 hours, 290 hours, 295 hours, 300 hours, 305 hours, 310 hours, 315 hours, 320 hours, 325 hours, 330 hours, 335 hours, 340 hours, 345 hours, 350 hours, 355 hours, or 360 hours.

In some embodiments, the glucocorticoid or pharmaceutically acceptable salt or ester thereof in the ropivacaine microspheres of the present invention is capable of sustained release for a duration of at least 24 hours, 25 hours, 26 hours, 27 hours, 28 hours, 29 hours, 30 hours, 31 hours, 32 hours, 33 hours, 34 hours, 35 hours, 36 hours, 37 hours, 38 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours, 61 hours, 62 hours, 63 hours, 64 hours, 65 hours, 66 hours, 67 hours, 68 hours, 69 hours, 70 hours, 71 hours, 72 hours, 73 hours, 74 hours, 75 hours, 76 hours, 77 hours, 78 hours, 79 hours, 80 hours, 81 hours, 82 hours, 83 hours, 84 hours, 85 hours, 86 hours, 87 hours, 88 hours, 89 hours, 90 hours, 91 hours, 92 hours, 93 hours, 94 hours, 95 hours, 96 hours, 97 hours, 98 hours, 99 hours or 100 hours.

In another aspect, the present invention relates to a ropivacaine microsphere, which comprises ropivacaine or a pharmaceutically acceptable salt thereof, at least one glucocorticoid or a pharmaceutically acceptable salt or ester thereof, and at least one biocompatible and biodegradable sustained-release material in an effective amount for extending the release of a drug from a preparation. Wherein the ropivacaine or the pharmaceutically acceptable salt thereof is capable of sustained release for a duration of at least 48 hours, and the glucocorticoid or the pharmaceutically acceptable salt or ester thereof is capable of sustained release for a duration of at least 24 hours.

In some embodiments, ropivacaine or pharmaceutically acceptable salt thereof in the ropivacaine microspheres of the present invention is capable of sustained release for a duration of at least 48 hours, 50 hours, 52 hours, 54 hours, 56 hours, 58 hours, 60 hours, 62 hours, 64 hours, 66 hours, 68 hours, 70 hours, 72 hours, 74 hours, 76 hours, 78 hours, 80 hours, 82 hours, 84 hours, 86 hours, 88 hours, 90 hours, 92 hours, 94 hours, 96 hours, 98 hours, 100 hours, 102 hours, 104 hours, 106 hours, 108 hours, 110 hours, 112 hours, 114 hours, 116 hours, 118 hours, 120 hours, 122 hours, 124 hours, 126 hours, 128 hours, 130 hours, 132 hours, 134 hours, 136 hours, 138 hours, 140 hours, 142 hours, 144 hours, 146 hours, 148 hours, 150 hours, 152 hours, 154 hours, 156 hours, 158 hours, 160 hours, 162 hours, 164 hours, 166 hours, 168 hours, 170 hours, 172 hours, 174 hours, 176 hours, 178 hours, 180 hours, 182 hours, 184 hours, 186 hours, 188 hours, 190 hours, 195 hours, 200 hours, 205 hours, 210 hours, 215 hours, 220 hours, 225 hours, 230 hours, 235 hours, 240 hours, 245 hours, 250 hours, 255 hours, 260 hours, 265 hours, 270 hours, 275 hours, 280 hours, 285 hours, 290 hours, 295 hours, 300 hours, 305 hours, 310 hours, 315 hours, 320 hours, 325 hours, 330 hours, 335 hours, 340 hours, 345 hours, 350 hours, 355 hours, or 360 hours.

In some embodiments, the glucocorticoid or pharmaceutically acceptable salt or ester thereof in the ropivacaine microspheres of the present invention is capable of sustained release for a duration of at least 24 hours, 25 hours, 26 hours, 27 hours, 28 hours, 29 hours, 30 hours, 31 hours, 32 hours, 33 hours, 34 hours, 35 hours, 36 hours, 37 hours, 38 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours, 61 hours, 62 hours, 63 hours, 64 hours, 65 hours, 66 hours, 67 hours, 68 hours, 69 hours, 70 hours, 71 hours, 72 hours, 73 hours, 74 hours, 75 hours, 76 hours, 77 hours, 78 hours, 79 hours, 80 hours, 81 hours, 82 hours, 83 hours, 84 hours, 85 hours, 86 hours, 87 hours, 88 hours, 89 hours, 90 hours, 91 hours, 92 hours, 93 hours, 94 hours, 95 hours, 96 hours, 97 hours, 98 hours, 99 hours or 100 hours.

In another aspect, the present invention relates to a ropivacaine microsphere, which, in terms of mass percentage, comprise 40-72% of ropivacaine or a pharmaceutically acceptable salt thereof, 0.1-3% of a glucocorticoid or a pharmaceutically acceptable salt or ester thereof, 20-65% of a biodegradable sustained-release material, and 0-15% of a porogen.

In some embodiments, the ropivacaine microspheres, in terms of mass percentage, comprise 44-69% of ropivacaine or the pharmaceutically acceptable salt thereof, 0.1-2.5% of a glucocorticoid or the pharmaceutically acceptable salt or ester thereof, 25-60% of a biodegradable sustained-release material, and 0-10% of a porogen.

In some embodiments, the ropivacaine microspheres, in terms of mass percentage, comprise 46-67% of ropivacaine or the pharmaceutically acceptable salt thereof, 0.1-2% of a glucocorticoid or the pharmaceutically acceptable salt or ester thereof, 30-56% of a biodegradable sustained-release material, and 0-8% of a porogen.

In some embodiments, the ropivacaine microspheres, in terms of mass percentage, comprise 46-67% of ropivacaine or the pharmaceutically acceptable salt thereof, 0.1-1.6% of a glucocorticoid or the pharmaceutically acceptable salt or ester thereof, 32-54% of a biodegradable sustained-release material, and 0-6% of a porogen.

On the other hand, the present invention relates to a method for preparing ropivacaine microspheres, which comprises the following steps:
(1) Dissolving ropivacaine or a pharmaceutically acceptable salt thereof, at least one glucocorticoid or a pharmaceutically acceptable salt or ester thereof, at least one biocompatible and biodegradable sustained-release material in an effective amount for extending the release of a drug from a preparation, and optional polyethylene glycol in an organic solvent to obtain an oil phase;
(2) Preparing a 1% polyvinyl alcohol aqueous solution and adjusting its pH to alkaline to obtain an aqueous phase;
(3) Preparing a 0.1% polyvinyl alcohol aqueous solution and adjusting its pH to alkaline to obtain a hardening solution; and
(4) Mixing the oil phase and the aqueous phase to prepare a primary emulsion, adding the primary emulsion to the hardening solution, stirring to remove the organic solvent, then collecting, washing, and drying.

In some embodiments, exemplary examples of organic solvents that can be used in the present invention include, but are not limited to, dichloromethane, ethyl acetate, acetone, chloroform, tetrahydrofuran, or mixtures thereof.

In some embodiments, exemplary examples of methods for preparing primary emulsion that can be used in the present invention include, but are not limited to, shearing, stirring, or static mixing.

In another aspect, the present invention relates to a method for local administration to provide long-acting analgesia or nerve block, which comprises administering ropivacaine microspheres. The microspheres comprise ropivacaine or a pharmaceutically acceptable salt thereof, at least one glucocorticoid or a pharmaceutically acceptable salt or ester thereof, and at least one biocompatible and biodegradable sustained-release material in an effective amount for extending the release of a drug from a preparation.

In some embodiments, for the microspheres that can be used in the drug delivery method of the present invention, when the cumulative release amount of ropivacaine or pharmaceutically acceptable salt thereof reaches 10%, 20%, 30%, 40%, 50%, 60%, 70%, or 80%, the sum of the differences between the cumulative release amounts of the glucocorticoid or the pharmaceutically acceptable salt or ester thereof and ropivacaine or the pharmaceutically acceptable salt thereof is not less than 105%, 110%, 115%, 120%, 125%, 130%, 135%, 140%, 145%, 150%, 155%, 160%, 165%, 170%, 175%, 180%, 185%, 190%, 195%, or 200%.

In some embodiments, for the microspheres that can be used in the drug delivery method of the present invention, wherein the cumulative release amount of ropivacaine or pharmaceutically acceptable salt thereof over 24 hours is not less than 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64% or 65%.

In some embodiments, for the microspheres that can be used in the drug delivery method of the present invention, the time required for the cumulative release of 80% of ropivacaine or the pharmaceutically acceptable salt thereof is at least 1.7 times, at least 1.8 times, at least 1.9 times, at least 2.0 times, at least 2.1 times, at least 2.2 times, at least 2.3 times, at least 2.4 times, at least 2.5 times, at least 2.6 times, at least 2.7 times, at least 2.8 times, at least 2.9 times, at least 3.0 times, at least 3.1 times, at least 3.2 times, at least 3.3 times, at least 3.4 times, at least 3.5 times, at least 3.6 times, at least 3.7 times, at least 3.8 times, at least 3.9 times, at least 4.0 times, at least 4.1 times, at least 4.2 times, at least 4.3 times, at least 4.4 times, at least 4.5 times, at least 4.6 times, at least 4.7 times, at least 4.8 times, at least 4.9 times, or at least 5.0 times the time required for the cumulative release of 80% of a glucocorticoid or the pharmaceutically acceptable salt or ester thereof.

In some embodiments, for the microspheres that can be used in the drug delivery method of the present invention, wherein the ropivacaine or pharmaceutically acceptable salt thereof is capable of sustained release for a duration of at least 48 hours, 50 hours, 52 hours, 54 hours, 56 hours, 58 hours, 60 hours, 62 hours, 64 hours, 66 hours, 68 hours, 70 hours, 72 hours, 74 hours, 76 hours, 78 hours, 80 hours, 82 hours, 84 hours, 86 hours, 88 hours, 90 hours, 92 hours, 94 hours, 96 hours, 98 hours, 100 hours, 102 hours, 104 hours, 106 hours, 108 hours, 110 hours, 112 hours, 114 hours, 116 hours, 118 hours, 120 hours, 122 hours, 124 hours, 126 hours, 128 hours, 130 hours, 132 hours, 134 hours, 136 hours, 138 hours, 140 hours, 142 hours, 144 hours, 146 hours, 148 hours, 150 hours, 152 hours, 154 hours, 156 hours, 158 hours, 160 hours, 162 hours, 164 hours, 166 hours, 168 hours, 170 hours, 172 hours, 174 hours, 176 hours, 178 hours, 180 hours, 182 hours, 184 hours, 186 hours, 188 hours, 190 hours, 195 hours, 200 hours, 205 hours, 210 hours, 215 hours, 220 hours, 225 hours, 230 hours, 235 hours, 240 hours, 245 hours, 250 hours, 255 hours, 260 hours, 265 hours, 270 hours, 275 hours, 280 hours, 285 hours, 290 hours, 295 hours, 300 hours, 305 hours, 310 hours, 315 hours, 320 hours, 325 hours, 330 hours, 335 hours, 340 hours, 345 hours, 350 hours, 355 hours or 360 hours.

In some embodiments, for the microspheres that can be used in the drug delivery method of the present invention, wherein the glucocorticoid or pharmaceutically acceptable salt or ester thereof is capable of sustained release for a duration of at least 24 hours, 25 hours, 26 hours, 27 hours, 28 hours, 29 hours, 30 hours, 31 hours, 32 hours, 33 hours, 34 hours, 35 hours, 36 hours, 37 hours, 38 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours, 61 hours, 62 hours, 63 hours, 64 hours, 65 hours, 66 hours, 67 hours, 68 hours, 69 hours, 70 hours, 71 hours, 72 hours, 73 hours, 74 hours, 75 hours, 76 hours, 77 hours, 78 hours, 79 hours, 80 hours, 81 hours, 82 hours, 83 hours, 84 hours, 85 hours, 86 hours, 87 hours, 88 hours, 89 hours, 90 hours, 91 hours, 92 hours, 93 hours, 94 hours, 95 hours, 96 hours, 97 hours, 98 hours, 99 hours or 100 hours.

In some embodiments, the microspheres of the present invention provide long-acting analgesia or nerve block with a duration of at least about 1 day, 2 days, 3 days, 4 days, 5 days, 6 days, 7 days, or longer.

In some embodiments, the ropivacaine or pharmaceutically acceptable salt thereof, and the glucocorticoid or pharmaceutically acceptable salt or ester thereof, in the microspheres of the present invention, are released at a specific ratio or rate, providing a more prolonged analgesic or nerve block effect.

In the following text, the present invention will be explained in detail through the following examples to facilitate a better understanding of its various aspects and advantages. However, it should be understood that the following examples are non-limiting and are provided solely for illustrative purposes of some embodiments of the present invention.

### [Example]

The reagents and equipment used in the examples of the present invention are conventional and commercially available. For example:

**Table 1: Sources of raw materials and excipients**

| Name of raw materials and excipients | Manufacturer |
|---|---|
| Ropivacaine | Shandong Mingfeng Pharmaceutical Technology Co., Ltd |
| Dexamethasone acetate | Shanghai Macklin Biochemical Technology Co., Ltd |
| Poly(lactide-co-glycolide) | Ashland (China) Investment Co., Ltd |
| Polyethylene glycol | Croda Chemicals (Shanghai) Co., Ltd |

**Table 2 Main experimental instruments and equipment**

| Main instruments and equipment | manufacturer | model |
|---|---|---|
| Electronic balance | Mettler Toledo Technology (China) Co., Ltd | ME4001/02 |
| Electronic balance | Mettler Toledo Technology (China) Co., Ltd | ME4001/02 |
| Electronic balance | Mettler Toledo Technology (China) Co., Ltd | ML204T |
| Shear disperser | IKA Instrument and Equipment Co., Ltd | T50 |
| Digital thermometer | Yuanhao Instrument Co., Ltd | YH-101 |
| Digital display top-mounted agitator | IKA Instrument and Equipment Co., Ltd | EURO-ST40 KLCDS025 |
| Immersion refrigerated and heating bath circulators | Hangzhou XUTEMP Technology Co., Ltd | XT5618-B24-R30C |
| Peristaltic pump | Cole-Parmer Instrument Company | MASCERFLEX FLS |

The formulation of ropivacaine-dexamethasone acetate microspheres in Examples 1-6 is shown in Table 3.

**Table 3**

| Composition | Example 1 | | Example 2 | | Example 3 | | Example 4 | | Example 5 | | Example 6 | |
|---|---|---|---|---|---|---|---|---|---|---|---|---|
| | Feeding amount/ g | Percentag e/% | Feeding amount/ g | Percenta ge / % | Feeding amount/ g | Percenta ge / % | Feeding amount/g | Percenta ge / % | Feeding amount/g | Percentage / % | Feeding amount/g | Percentage / % |
| Ropivacaine | 1.10 | 53.92 | 2.17 | 55.22 | 1.08 | 54.54 | 2.16 | 55.24 | 2.16 | 56.10 | 4.32 | 55.24 |
| Dexamethaso ne acetate | 0.03 | 1.47 | 0.01 | 0.25 | 0.03 | 1.52 | 0.01 | 0.26 | 0.01 | 0.26 | 0.01 | 0.13 |
| Poly(lactic-c o-glycolic acid) 75:25 | 0.79 | 38.73 | 1.59 | 40.46 | 0.79 | 39.90 | 1.58 | 40.41 | 1.59 | 41.30 | 3.16 | 40.41 |
| Polyethylene glycol 4000 | 0.12 | 5.88 | 0.16 | 4.07 | 0.08 | 4.04 | 0.16 | 4.09 | 0.09 | 2.34 | 0.33 | 4.22 |
| Dichlorometh ane* | 10.0 | N/A | 20.2 | N/A | 10 | N/A | 20.2 | N/A | 20.0 | N/A | 40.3 | N/A |
| Polyvinyl alcohol* | 2.60 | N/A | 5.2 | N/A | 2.60 | N/A | 5.20 | N/A | 5.20 | N/A | 10.4 | N/A |
| Purified water* | 1697.4 | N/A | 3394.8 | N/A | 1697.7 | N/A | 3394.8 | N/A | 3395.2 | N/A | 6779.6 | N/A |
| Total | 2.04 | 100.0 | 3.93 | 100.0 | 1.98 | 100.0 | 3.91 | 100.0 | 3.85 | 100.0 | 7.82 | 100.0 |

| | | | | | | | | | | | | |
|---|---|---|---|---|---|---|---|---|---|---|---|---|
| Note: *It is removed during the process, and its usage is not counted in the intermediate or finished products. | | | | | | | | | | | | |

The formulation of ropivacaine-dexamethasone acetate microspheres in Examples 7-10 is shown in Table 4.

**Table 4**

| Composition | Example 7 | | Example 8 | | Example 9 | | Example 10 | |
|---|---|---|---|---|---|---|---|---|
| | Feeding amount/g | Percentage /% | Feeding amount/g | Percentage / % | Feeding amount/g | Percentage / % | Feeding amount/g | Percentage /% |
| Ropivacaine | 3.84 | 65.19 | 3.84 | 66.01 | 7.52 | 60.64 | 6.00 | 55.25 |
| Dexamethasone acetate | 0.07 | 1.19 | 0.07 | 1.20 | 0.13 | 1.05 | 0.11 | 1.01 |
| Poly(lactic-co-glycolic acid) 75:25 | 1.89 | 32.09 | 1.91 | 32.82 | 4.75 | 38.31 | 4.75 | 43.74 |
| Polyethylene glycol 4000 | 0.09 | 1.53 | / | / | / | / | / | / |
| Dichloromethane* | 24.1 | N/A | 24.1 | N/A | 60 | N/A | 60 | N/A |
| Polyvinyl alcohol* | 9.45 | N/A | 9.45 | N/A | 15.42 | N/A | 15.77 | N/A |
| Purified water* | 4391.95 | N/A | 4391.95 | N/A | 10166.18 | N/A | 10200.83 | N/A |
| Total | 5.89 | 100.0 | 5.82 | 100.0 | 12.40 | 100.0 | 10.86 | 100.0 |

| | | | | | | | | |
|---|---|---|---|---|---|---|---|---|
| Note: *It is removed during the process, and its usage is not counted in the intermediate or finished products. | | | | | | | | |

The formulation of ropivacaine-dexamethasone acetate microspheres in Comparative Examples 1-4 is shown in Table 5.

**Table 5**

| Composition | Comparative Example 1 | | Comparative Example 2 | | Comparative Example 3 | | Comparative Example 4 | |
|---|---|---|---|---|---|---|---|---|
| | Feeding amount/g | Percentage /% | Feeding amount/g | Percentage /% | Feeding amount/g | Percentage /% | Feeding amount/g | Percentag e/% |
| Ropivacaine | 0.46 | 32.86 | 0.79 | 48.77 | 3.84 | 66.10 | 3.84 | 65.20 |
| Dexamethasone acetate | 0.03 | 2.14 | 0.03 | 1.85 | 0.07 | 1.20 | 0.07 | 1.19 |
| Poly(lactic-co-glycolic acid) 75:25 | 0.79 | 56.43 | 0.80 | 49.38 | 1.90 | 32.70 | 1.98 | 33.61 |
| Polyethylene glycol 4000 | 0.12 | 8.57 | / | / | / | / | / | / |
| Dichloromethane* | 10 | N/A | 10 | N/A | 24.2 | N/A | 24.1 | N/A |
| Polyvinyl alcohol* | 2.60 | N/A | 2.60 | N/A | 9.45 | N/A | 9.45 | N/A |
| Purified water* | 1697.3 | N/A | 1697.4 | N/A | 4391.95 | N/A | 4391.95 | N/A |
| Total | 1.40 | 100.0 | 1.62 | 100.0 | 5.81 | 100.0 | 5.89 | 100.0 |

| | | | | | | | | |
|---|---|---|---|---|---|---|---|---|
| Note: *It is removed during the process, and its usage is not counted in the intermediate or finished products. | | | | | | | | |

The formulation of ropivacaine-dexamethasone acetate microspheres in Examples 11-12 is shown in Table 6.

**Table 6**

| Composition | Example 11 | | Example 12 | |
|---|---|---|---|---|
| | Feeding amount/g | Percentage / % | Feeding amount/g | Percentage / % |
| Ropivacaine | 5.02 | 60.77 | 2.73 | 46.04 |
| Dexamethasone | 0.08 | 0.97 | 0.04 | 0.67 |
| Poly(lactic-co-glycolic acid) 75:25 | 3.16 | 38.26 | 3.16 | 53.29 |
| Polyethylene glycol 4000 | / | / | / | / |
| Dichloromethane* | 40.1 | N/A | 40 | N/A |
| Polyvinyl alcohol* | 11.84 | N/A | 11.84 | N/A |
| Purified water* | 6932.06 | N/A | 6932.56 | N/A |
| Total | 8.26 | 100.0 | 5.93 | 100.0 |

| | | | | |
|---|---|---|---|---|
| Note: *It is removed during the process, and its usage is not counted in the intermediate or finished products. | | | | |

**Table 7 Models and viscosities of poly(lactic-co-glycolic acid)**

| | Examples 1 to 12 | Comparative Example 1 | Comparative Example 2 | Comparative Example 3 | Comparative Example 4 |
|---|---|---|---|---|---|
| Model | Ashland/7525 | Merck/DLG752A: Evonik/DLG4A=3: 1 | Ashland/ 7525 2A | Evonik/ 75252CA | Evonik/ 5050 2A |
| Viscosity | 0.12 | 0.215 | 0.12 | 0.15 | 0.21 |

Preparation methods for Examples 1-6 and Comparative Examples 1-2:
The prescribed amounts of ropivacaine, dexamethasone acetate, poly(lactic-co-glycolic acid), and polyethylene glycol were weighed and dissolved in dichloromethane to obtain an oil phase. An appropriate amount of PVA (polyvinyl alcohol) was weighed and dissolved in water to prepare a 1% (w/w) solution, and the pH was adjusted to alkaline to obtain an aqueous phase, which was then cooled to 15°C. The remaining PVA from the prescription was dissolved in water to prepare a 0.1% (w/w) solution, and the pH was adjusted to alkaline to obtain a hardening solution, which was then cooled to 15°C. Under shear conditions, the oil phase was added to the aqueous phase for emulsification, followed by the slow addition of the hardening solution. The organic solvent was removed by stirring in a water bath with air blowing. The resulting microspheres were collected, washed with water to remove residual PVA, dried, and sieved to obtain the final microspheres.

Preparation methods for Examples 7-12 and Comparative Examples 3-4:
The prescribed amounts of ropivacaine, dexamethasone or dexamethasone acetate, poly(lactic-co-glycolic acid) were weighed and dissolved in dichloromethane to obtain an oil phase. An appropriate amount of PVA was weighed and dissolved in water to prepare a 1% (w/w) solution, and the pH was adjusted to alkaline to obtain an aqueous phase, which was then cooled to 15°C. The remaining PVA from the prescription was dissolved in water to prepare a 0.1% (w/w) solution, the pH was adjusted to alkaline to obtain a hardening solution, which was then cooled to 15°C, and water bath stirring was initiated. The static mixer was connected. The oil phase and the aqueous phase were linked to the inlet port of the static mixer, and the feeding rates were controlled via peristaltic pumps. The outlet port of the static mixer was connected to the hardening solution. The peristaltic pump controlling the aqueous phase was started first to allow the aqueous phase to enter the hardening solution through the static mixer. Immediately afterward, the peristaltic pump controlling the oil phase was started. After the oil phase was completely introduced into the hardening solution, the aqueous phase tube was removed from the aqueous phase. The peristaltic pump was kept running to allow all liquid within the static mixer to be expelled, after which the peristaltic pump was turned off. The organic solvent in the hardening solution was removed by air blowing, followed by filtration to collect the microspheres. The microspheres were washed with water to remove residual PVA, dried, and sieved to obtain the final microspheres.

### Example 13: Particle size detection of microspheres

**Table 8 Particle size detection results of microspheres**

| | D₁₀ (µm) | D₅₀ (µm) | D₉₀ (µm) |
|---|---|---|---|
| Example 3 | 27.7 | 36.7 | 48.9 |
| Example 7 | 20.5 | 33.8 | 54.5 |
| Example 9 | 19.6 | 31.7 | 49.5 |
| Example 12 | 20.5 | 33.8 | 54.5 |

### Example 14: Determination of in vitro release curve

### Release degree determination:

Preparation method of release medium (pH 7.4 phosphate buffer solution with): 34 g of potassium dihydrogen phosphate, 1 g of polysorbate 80, 1 g of polysorbate 20, 7 g of sodium hydroxide, and 1 g of sodium azide were weighed and added to water. The mixture was stirred until complete dissolution, made up to a final volume of 5 L with water, and the pH value was adjusted to 7.4 using a small amount of sodium hydroxide solution. Finally, the solution was sterilized by moist heat at 121°C for 15 minutes.

Release degree determination method: The water bath was set at 37°C and left to stand. A phosphate buffer solution with pH 7.4 was used as the release medium, with a medium volume of 100 mL. 20 mg of each test sample (microspheres prepared in Examples 1-12 and Comparative Examples 1-4) was weighed into a blue-cap bottle, to which 100 mL of the release medium was added. The bottle was then allowed to stand in a 37°C water bath. Samples (1 mL) were withdrawn at 1 h, 4 h, 8 h, 24 h, 48 h, 72 h, 120 h, 168 h, and 216 h, with 1 mL of fresh release medium being replenished after each sampling. The mixture was centrifuged (8000 rpm, 10 min), and 0.5 mL of the supernatant was transferred into an HPLC vial for release determination. The cumulative release degrees of ropivacaine and dexamethasone acetate (or dexamethasone) at different time points were calculated separately.

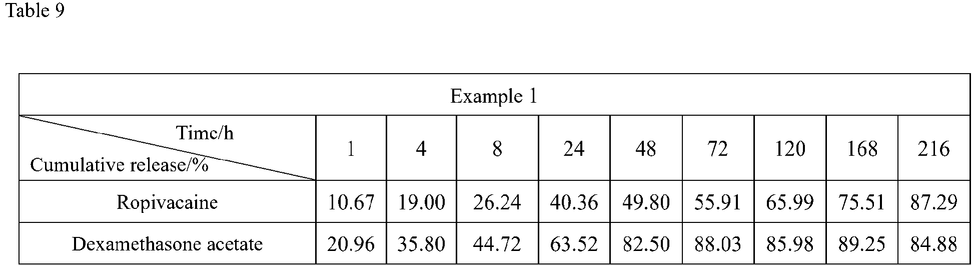

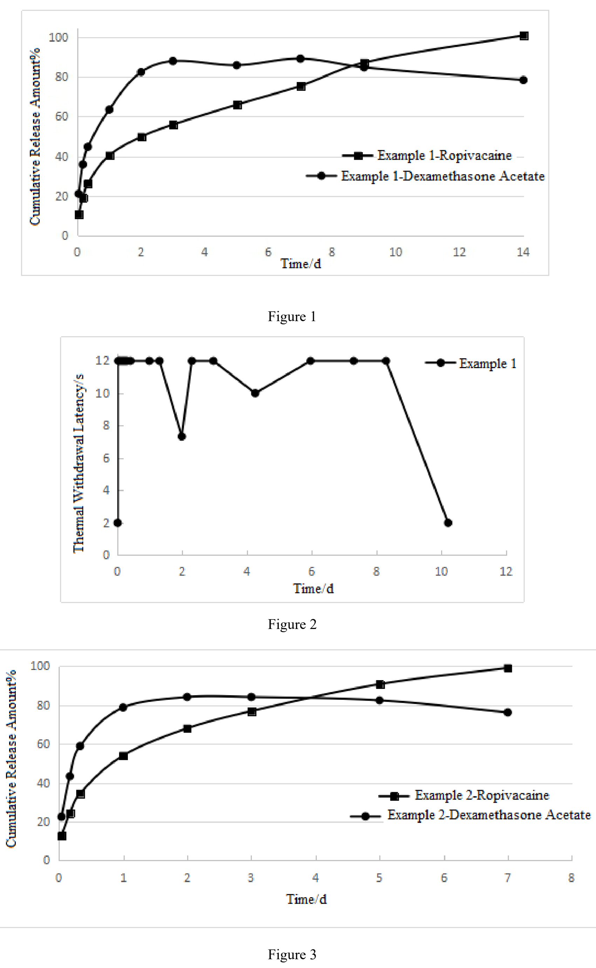

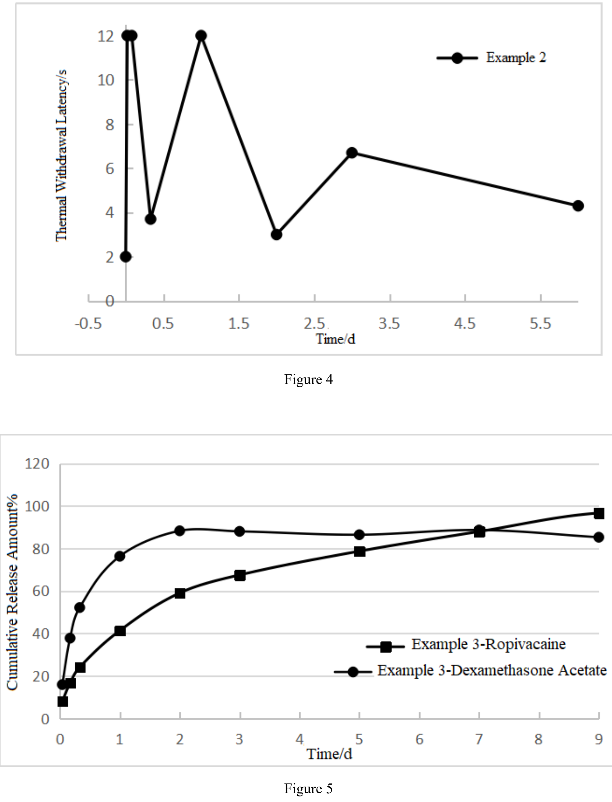

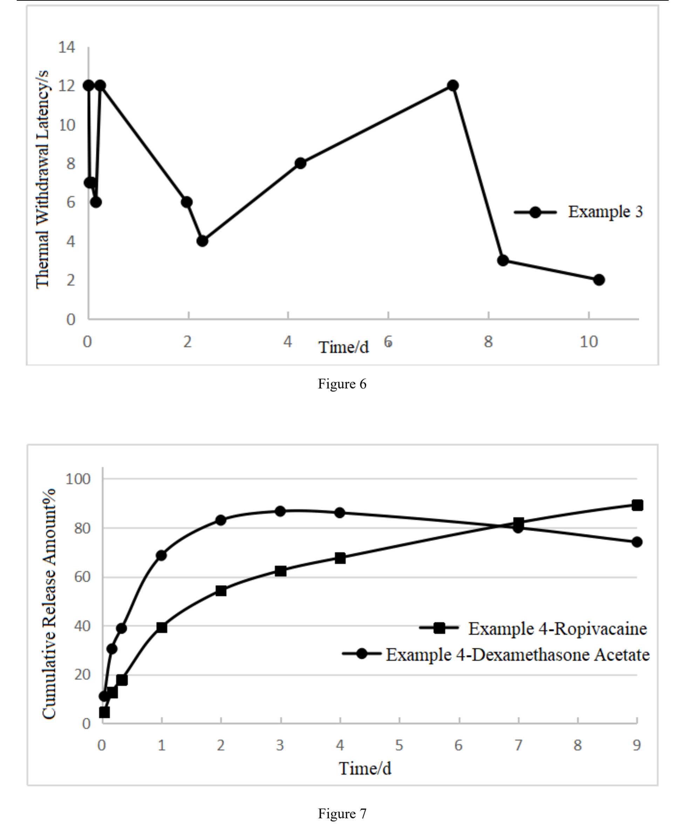

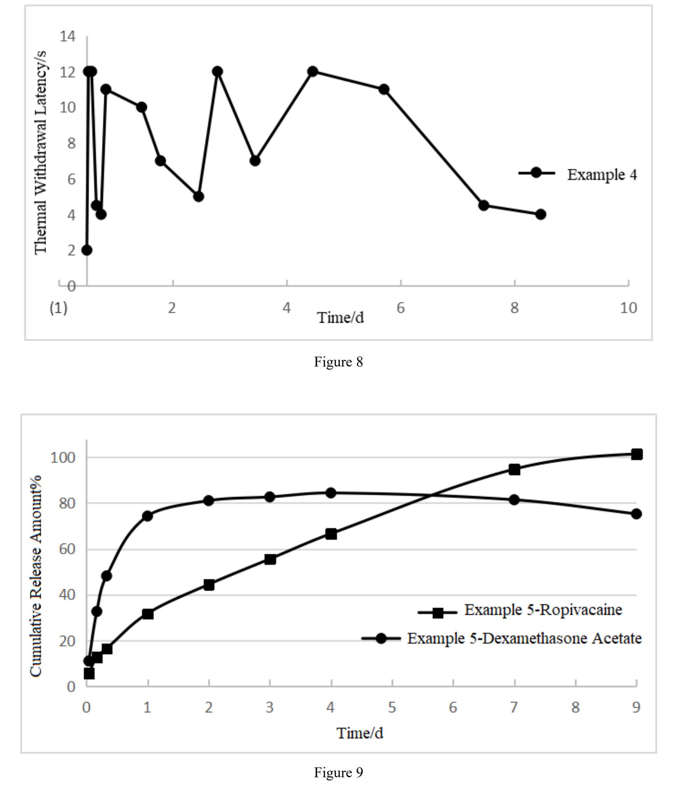

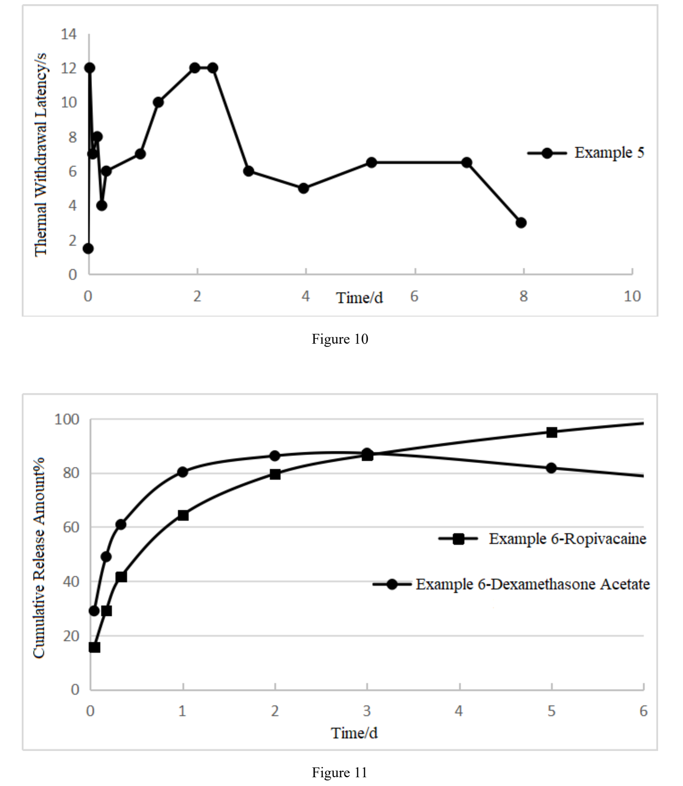

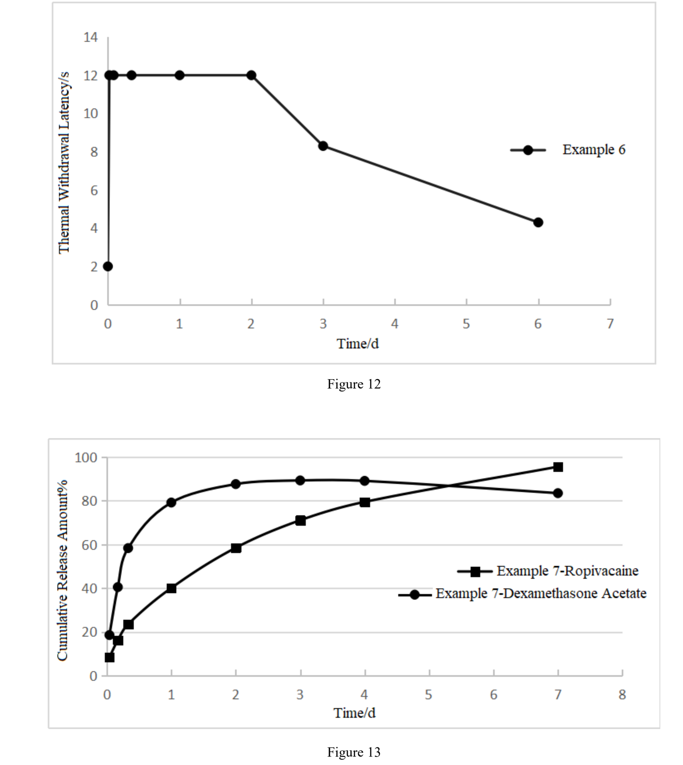

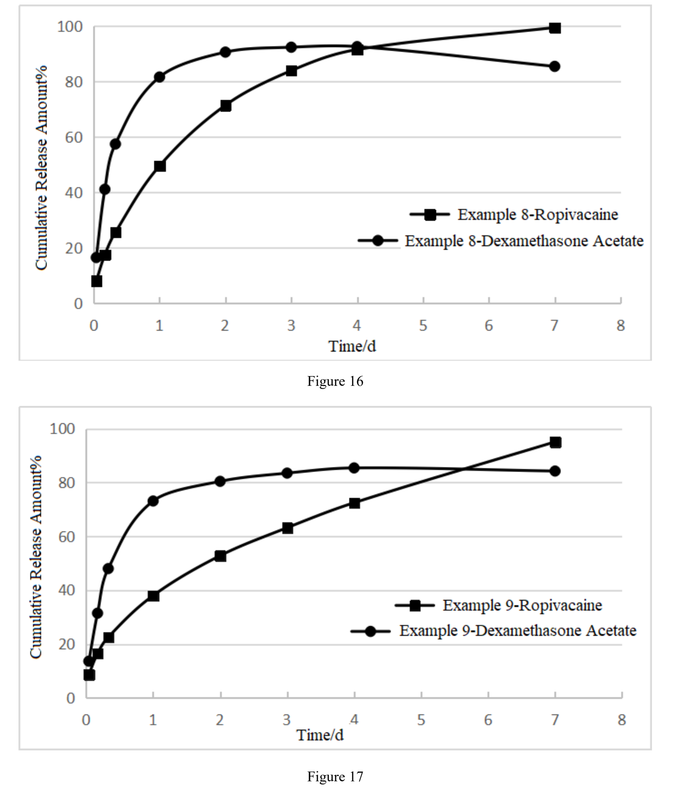

Figures 1, 3, 5, 7, 9, 11, 13, and 16-18 depict the cumulative release curves of ropivacaine-dexamethasone acetate microspheres in Examples 1-10 of the present invention, respectively.

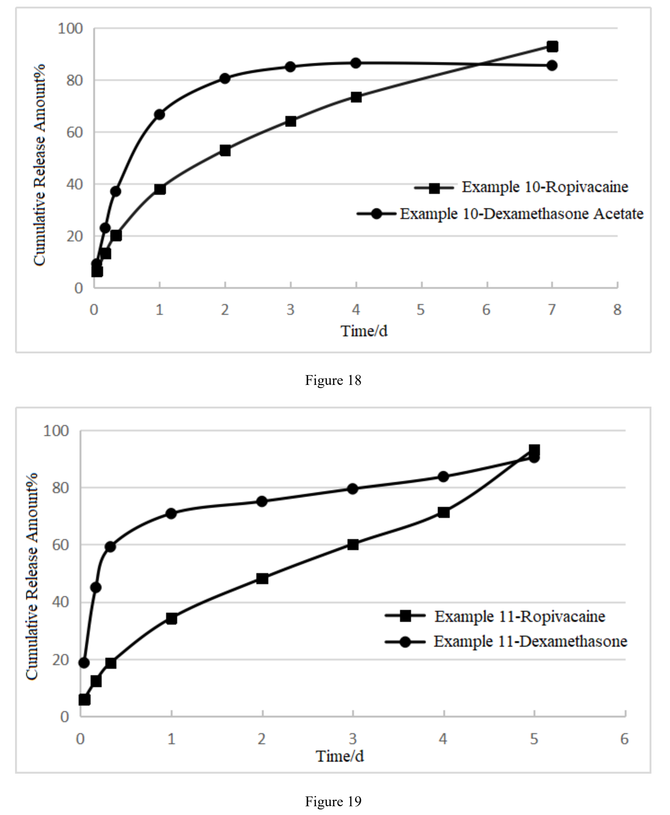

Figures 19-20 depict the cumulative release curves of ropivacaine-dexamethasone microspheres in Examples 11-12 of the present invention, respectively.

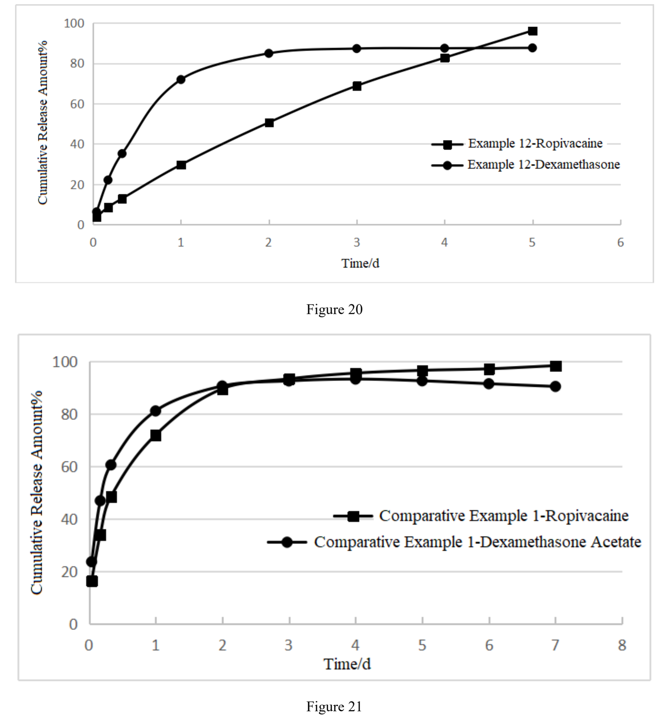

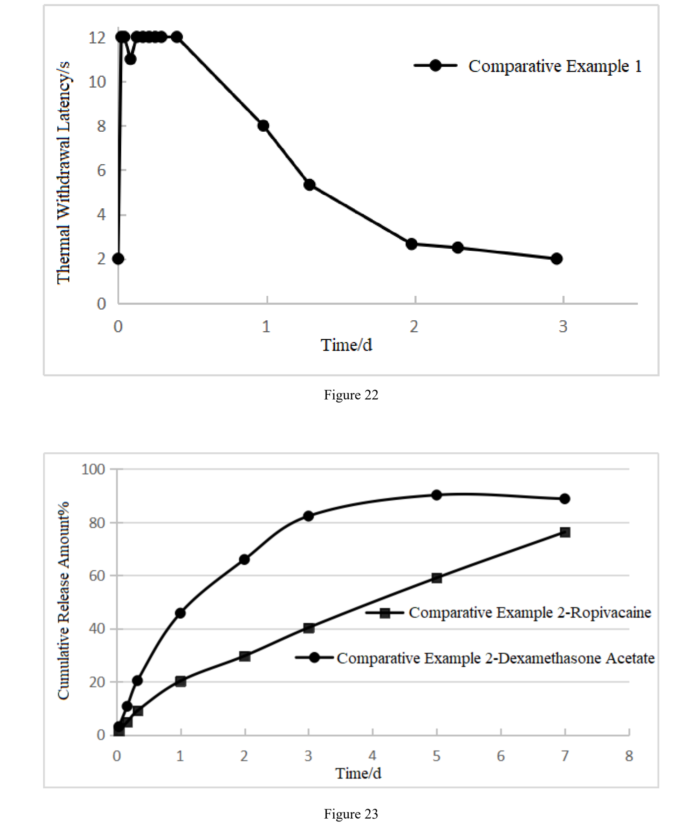

Figures 21 and 23 depict the cumulative release curves of ropivacaine-dexamethasone acetate microspheres in Comparative Examples 1-2 of the present invention, respectively. It can be observed that the release rate of the microspheres in Comparative Example 1 is too fast, while the release rate of the microspheres in Comparative Example 2 is too slow.

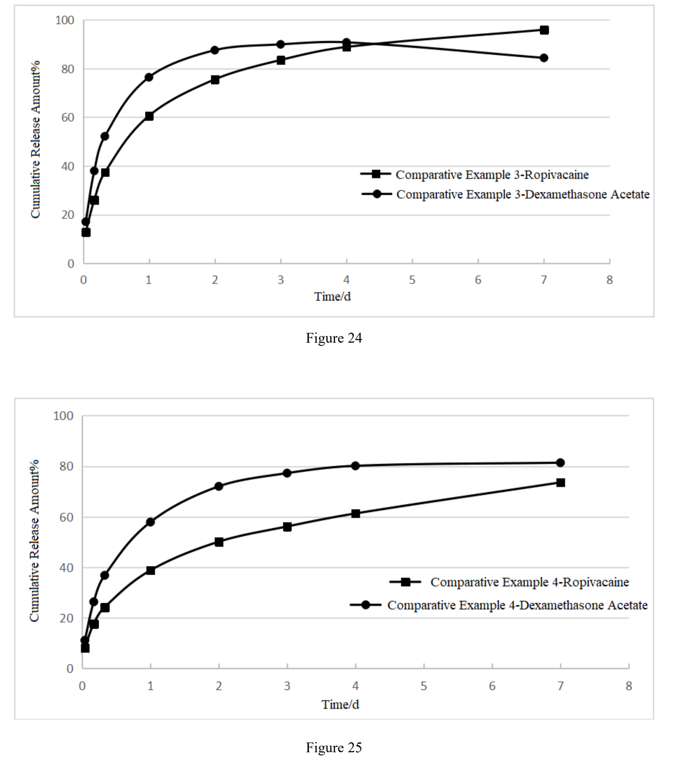

Figures 24 and 25 depict the cumulative release curves of ropivacaine-dexamethasone acetate microspheres in Comparative Example 3 and Comparative Example 4 of the present invention, respectively.

### Example 15: Pharmacodynamic evaluation of rat sciatic nerve block model

The microspheres prepared in Examples 1-6 and Comparative Examples 1-2 were subjected to local anesthesia testing using thermal delay to evaluate their effectiveness. Specifically, the duration of functional sciatic nerve block was tested by inducing a harmful thermal stimulus of approximately 56°C on the plantar surface of the paw, resulting in a delay in leg withdrawal response in rats. The normal delay for paw withdrawal is approximately 2 seconds, while "maximum sensory block" is characterized by a delay of approximately 12 seconds.

According to the body weight of rats, a microsphere suspension with a dose of 200 mg/kg (calculated as ropivacaine) was injected around the sciatic nerve on the inner thigh of the left hind limb. Thermal pain was assessed before administration and at 30 minutes, 2 hours, 4 hours, 8 hours, 24 hours, 1.5 days, 2 days, 3 days, 4 days, 5 days, 6 days, 7 days, and 9 days after administration. The time from rat contact with the hot plate to the onset of paw withdrawal was recorded as the thermal withdrawal latency (TWL). If no response was observed after 12 seconds, the contact was terminated, and a maximum thermal withdrawal latency of 12 seconds was assigned.

Figures 2, 4, 6, 8, 10, and 12 depict the thermal pain threshold curves of ropivacaine-dexamethasone acetate microspheres in Examples 1-6 of the present invention.

Figure 22 shows the thermal pain threshold curve of ropivacaine-dexamethasone acetate microspheres in Comparative Example 1. Due to the excessively fast release rate of the microspheres, they can only maintain analgesic effects for 1 day.

The ropivacaine-dexamethasone acetate microspheres in Comparative Example 2 did not take effect due to their excessively slow release.

### Example 16: Pharmacodynamic study of rat plantar incision pain model

The animals were subject to one week of adaptive feeding (including environmental adaptation training for mechanical pain and heat-sensitive pain conducted daily for the last three days). Prior to surgery, the mechanical pain threshold and heat-induced paw contraction threshold of all rats were measured. Rats were then grouped based on their mechanical pain threshold and heat-induced paw contraction threshold. Following grouping, modeling was performed. On the day of modeling, rats designated for modeling were anesthetized with inhaled isoflurane. The left hind paw was disinfected and draped. A 1 cm long longitudinal incision was made approximately 0.5 cm from the proximal end of the plantar surface. The plantar muscles were lifted with ophthalmic forceps and longitudinally incised, while preserving the integrity of their origins, insertions, and attachments. After hemostasis was achieved by compression, and prior to closing the incision, 100 µL of the test substance (microspheres prepared in Example 7) was injected subcutaneously into the plantar region. Fascia and skin were sutured with absorbable fine sutures to close the incision. The day of modeling was designated as D0.
(1) Paw Withdrawal Threshold (PWT) test: The test was conducted once before surgery and then at 2 hours, 4 hours, 24 hours, 48 hours, 72 hours, 120 hours, and 168 hours postoperatively. All rats were subjected to pain measurement using a Von Fery electronic pain detector. Positive responses were recorded when the animals exhibited behaviors such as paw licking, leg lifting, or avoidance.

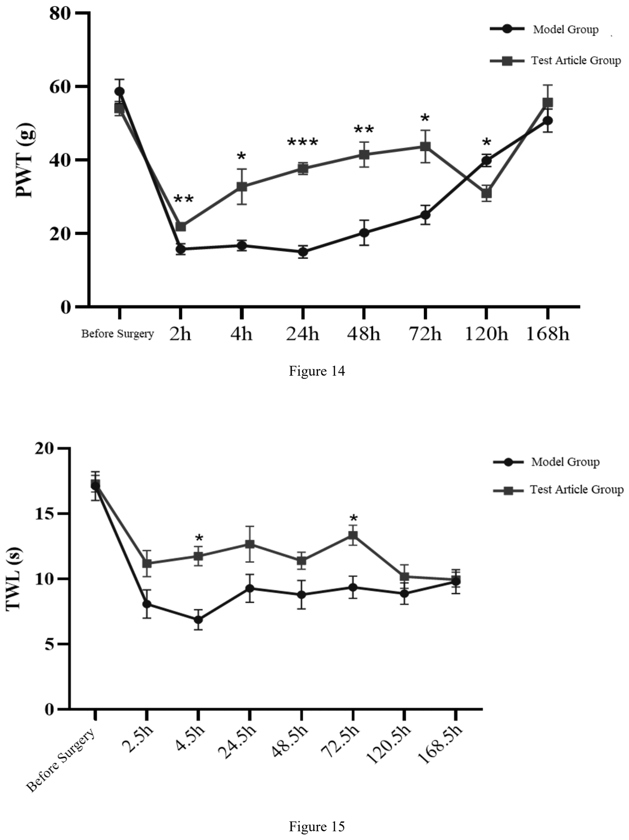

   Figure 14 shows the paw withdrawal threshold curve of ropivacaine-dexamethasone acetate microspheres in Example 7 of the present invention.
(2) Thermal Withdrawal Latency (TWL) Test: The test was conducted once before surgery and then at 2.5 hours, 4.5 hours, 24.5 hours, 48.5 hours, 72.5 hours, 120.5 hours, and 168.5 hours postoperatively. A thermal radiation source (radiation intensity 5%) was focused directly on the center of the wound. The thermal radiation source was automatically shut off when the rat exhibited a paw withdrawal response (lifting or licking the hind paw). The time interval from the onset of irradiation to the withdrawal response was recorded as TWL. To avoid thermal injury, once the radiation time exceeded 35 seconds, the radiation was stopped, and 35 seconds was used as the maximum pain threshold.

Figure 15 shows the thermal withdrawal latency curve of ropivacaine-dexamethasone acetate microspheres in Example 7 of the present invention.

In the present invention, relational terms such as "first" and "second" are merely used to distinguish one entity or operation from another, without necessarily requiring or implying any actual relationship or order between these entities or operations.

From the foregoing, it can be understood that although specific embodiments of the present invention have been described for illustrative purposes, various variations or improvements can be made by those skilled in the art without departing from the spirit and scope of the present invention. These variations or modifications should fall within the scope of the appended claims of the present invention.

## Claims

1. A ropivacaine microsphere, comprising ropivacaine or a pharmaceutically acceptable salt thereof, at least one glucocorticoid or a pharmaceutically acceptable salt or ester thereof, and at least one biocompatible and biodegradable sustained-release material in an effective amount for extending the release of a drug from a preparation, wherein the time required for the cumulative release of 80% of ropivacaine or the pharmaceutically acceptable salt thereof is at least 1.7 times the time required for the cumulative release of 80% of the glucocorticoid or the pharmaceutically acceptable salt or ester thereof.

2. The ropivacaine microsphere according to claim 1, wherein the cumulative release amount of ropivacaine or the pharmaceutically acceptable salt thereof over 24 hours is not less than 25%.

3. The ropivacaine microsphere according to claim 1 or 2, wherein ropivacaine or the pharmaceutically acceptable salt thereof is capable of sustained release for a duration of at least 48 hours.

4. The ropivacaine microsphere according to any one of claims 1 to 3, wherein the glucocorticoid or the pharmaceutically acceptable salt or ester thereof is capable of sustained release for a duration of at least 24 hours.

5. The ropivacaine microsphere according to any one of claims 1 to 4, wherein the glucocorticoid or the pharmaceutically acceptable salt or ester thereof is one or more selected from the group consisting of cortisone, hydrocortisone, prednisone, prednisolone, methylprednisolone, deflazacort, fludrocortisone, triamcinolone, betamethasone, dexamethasone, triamcinolone acetonide, methylprednisolone, paramethasone, beclomethasone, flunisolide, and pharmaceutically acceptable salts or esters thereof, preferably one or more selected from the group consisting of dexamethasone, dexamethasone acetate, dexamethasone sodium phosphate, dexamethasone palmitate, betamethasone, and betamethasone dipropionate.

6. A ropivacaine microsphere, comprising ropivacaine or a pharmaceutically acceptable salt thereof, at least one glucocorticoid or a pharmaceutically acceptable salt or ester thereof, and at least one biocompatible and biodegradable sustained-release material in an effective amount for extending the release of a drug from a preparation, wherein, when the cumulative release amount of ropivacaine or the pharmaceutically acceptable salt thereof reaches 10%, 20%, 30%, 40%, 50%, 60%, 70%, or 80%, the sum of the differences between the cumulative release amounts of the glucocorticoid or the pharmaceutically acceptable salt or ester thereof and ropivacaine or the pharmaceutically acceptable salt thereof is not less than 105%.

7. The ropivacaine microsphere according to claim 6, wherein the cumulative release amount of ropivacaine or the pharmaceutically acceptable salt thereof over 24 hours is not less than 25%.

8. The ropivacaine microsphere according to claim 6 or 7, wherein ropivacaine or the pharmaceutically acceptable salt thereof is capable of sustained release for a duration of at least 48 hours.

9. The ropivacaine microsphere according to claim 6 or 7, wherein the glucocorticoid or the pharmaceutically acceptable salt or ester thereof is capable of sustained release for a duration of at least 24 hours.

10. The ropivacaine microsphere according to claim 6, wherein the glucocorticoid or the pharmaceutically acceptable salt or ester thereof is one or more selected from the group consisting of cortisone, hydrocortisone, prednisone, prednisolone, methylprednisolone, deflazacort, fludrocortisone, triamcinolone, betamethasone, dexamethasone, triamcinolone acetonide, methylprednisolone, paramethasone, beclomethasone, betamethasone, flunisolide, and pharmaceutically acceptable salts or esters thereof.

11. A ropivacaine microsphere, comprising ropivacaine or a pharmaceutically acceptable salt thereof, at least one glucocorticoid or a pharmaceutically acceptable salt or ester thereof, and at least one biocompatible and biodegradable sustained-release material in an effective amount for extending the release of a drug from a preparation, wherein ropivacaine or the pharmaceutically acceptable salt thereof is capable of sustained release for a duration of at least 48 hours, and the glucocorticoid or the pharmaceutically acceptable salt or ester thereof is capable of sustained release for a duration of at least 24 hours.

12. The ropivacaine microsphere according to claim 11, wherein the cumulative release amount of ropivacaine or the pharmaceutically acceptable salt thereof over 24 hours is not less than 25%.

13. The ropivacaine microsphere according to claim 11 or 12, wherein the glucocorticoid or the pharmaceutically acceptable salt or ester thereof is one or more selected from the group consisting of cortisone, hydrocortisone, prednisone, prednisolone, methylprednisolone, deflazacort, fludrocortisone, triamcinolone, betamethasone, dexamethasone, triamcinolone acetonide, methylprednisolone, paramethasone, beclomethasone, betamethasone, flunisolide, and pharmaceutically acceptable salts or esters thereof.

14. A ropivacaine microsphere, wherein, by mass percentage, the ropivacaine microsphere comprises: 40-72% of ropivacaine or a pharmaceutically acceptable salt thereof, 0.1-3% of a glucocorticoid or a pharmaceutically acceptable salt or ester thereof, 20-65% of a biodegradable sustained-release material, and 0-15% of a porogen.

15. A method for preparing the ropivacaine microsphere according to any one of claims 1 to 14, comprising the following steps:
(1) dissolving ropivacaine or a pharmaceutically acceptable salt thereof, at least one glucocorticoid or a pharmaceutically acceptable salt or ester thereof, at least one biocompatible and biodegradable sustained-release material in an effective amount for extending the release of a drug from a preparation, and optional polyethylene glycol in an organic solvent to obtain an oil phase;
(2) preparing a 1% polyvinyl alcohol aqueous solution and adjusting its pH to alkaline to obtain an aqueous phase;
(3) preparing a 0.1% polyvinyl alcohol aqueous solution and adjusting its pH to alkaline to obtain a hardening solution; and
(4) mixing the oil phase and the aqueous phase to prepare a primary emulsion, adding the primary emulsion to the hardening solution, stirring to remove the organic solvent, then washing, collecting, and drying.

16. A method for local administration to provide long-acting analgesia or nerve block, comprising administering the ropivacaine microspheres according to any one of claims 1 to 14 to a subject in need.

17. The method according to claim 16, wherein the duration of analgesia or nerve block after first administration of the microspheres is at least about 1 day.
